# EUROPEAN PATENT SPECIFICATION

(11) **EP 2 446 915 B1**
(45) Date of publication and mention of the grant of the patent: **24.01.2018**
(21) Application number: 11186119.1
(22) Date of filing: 21.10.2011
(51) Int. Cl.: A61M 25/10, A61M 25/00, A61F 2/966, A61M 29/02, A61M 25/06

(54) **Introducer assembly and dilator tip therefor**
Inserteranordnung und Dilatatorspitze dafür
Ensemble d'introducteur et pointe de dilatateur correspondante

(30) Priority: 02.11.2010 GB 201018470
(43) Date of publication of application: 02.05.2012
(73) Proprietor: Cook Medical Technologies LLC, Bloomington, IN 47404 (US)
(72) Inventor: Rasmussen, Erik, DK-4200 Slagelse (DK)
(74) Representative: Georgiou, Sarah Caroline

(56) References cited:
- WO-A1-2010/078077
- US-A1- 2008 255 652
- US-A1- 2009 264 859

## Description

### Field of the Invention

The present invention relates to an introducer assembly for deploying an implantable medical device.

### Background of the Invention

Introducer assemblies are in common use for deploying endoluminally a variety of implantable medical devices. These assemblies are generally of an "over the wire" type, in which a guide wire is first passed through the vasculature of a patient up to an often beyond the site at which treatment is to be effected. The guide wire acts to guide the subsequent insertion of the introducer assembly into the patient, which assembly will carry a medical device to be implanted in the patient or otherwise apparatus such as catheters, angioplasty balloons and so on, to the treatment site. The introducer assembly, whether or not of the over the wire type, will generally include a sheath or catheter of a size able to hold the medical device or other equipment to be introduced, as well as a dilator tip at the distal end of the introducer assembly. The dilator tip is important in assisting in guiding the sheath and components held therewithin through the patient's vasculature and in seeking to ensure that the walls of the patient's vessels are not harmed or traumatised during the insertion procedure.

In the case of deployment of a medical device, the dilator tip, which is typically of a flexible material and of a diameter substantially the same as that of the sheath, must be passed beyond the treatment site so as to be able to position the medical device at the site itself. In most cases there is no issue presented with having the dilator tip pass beyond the treatment site as this in practice will lie within the patient's vasculature and then be retrieved with the sheath and other components once the medical device has been released from the introducer and deployed. In fact, in some instances the dilator tip is intentionally fed in a distal direction, that is forwards during the deployment procedure.

There are some circumstances, however, where it is disadvantageous to have the dilator tip positioned beyond the treatment site, particularly when this results in the dilator tip being moved into a delicate part of a patient's lumen or against or into an organ or other delicate site. A particular issue arises when it is desired to place a medical device very close to the heart, where there is a compromise between the proximity to the heart to which a device can be placed and having to pass the dilator tip beyond the aortic valve. In some cases it might not be disadvantageous to push the dilator tip through the heart valve but there are many instances where this is not desirable. A problem with current introducer systems is that if the dilator tip is to be kept clear of the aortic valve, there is a limit to the position in which the device can be positioned, in light of the length of the dilator tip. Thus, either the device cannot be positioned close enough to the aortic valve or the dilator tip has to breach the valve.

US 2008255652 discloses an introducer assembly for deploying a stent-graft in a curved vessel including an expandable tip.

WO2010078077 discloses an introducer for deploying a deployable device. The introducer has a balloon tip.

US 2009/0264859 describes a catheter including an expandable distal tip. The tip may be expanded to dislodge the catheter from an obstacle or to keep the catheter from becoming engaged in an obstacle.

### Brief Description of the Invention

The present invention seeks to provide an improved introducer assembly and an improved method of deploying implantable medical devices.

According to the present invention, there is provided an introducer assembly including: a dilator tip; a sheath having a diameter; an implantable medical device located within said sheath and providing a lumen therewithin when located in said sheath; wherein the dilator tip is formed with a radially expandable and contractible element, wherein said element has an expanded diameter substantially equal to the diameter of the sheath and a contracted diameter smaller than the diameter of the lumen of said implantable medical device, said dilator tip being retractable when in the contracted condition into said lumen.

This structure enables the use of a dilator tip with an introducer assembly and also enables the positioning of the sheath and of the implantable medical device right at the distal extremity of the assembly, by the ability withdraw the dilator tip into the sheath once the assembly has reached the treatment site. In this manner, it is possible to extend the assembly and the medical device right up to a part of a patient's anatomy which is not to be touched, a feature not possible with prior art devices. The assembly taught herein therefore can avoid the above-mentioned compromises of prior art devices.

It is to be understood that by an expanded diameter of the dilator tip which is substantially equal to the diameter of the sheath this may be the same as, smaller than or larger than the diameter of the sheath. However, the dilator tip has a diameter which is close to the diameter of the sheath, typically an expanded diameter which is smaller than the expanded diameter of the medical device smaller than the diameter of the lumen. It is also to be understood that in embodiments where the dilator tip is larger than the diameter of the sheath, it is not substantially greater than the diameter of the sheath and retains the function and characteristics of a dilator tip and not of a different component such as a balloon.

Preferably, the element is a balloon, said balloon having an inflated diameter substantially no greater than the diameter of the sheath. In an embodiment, the balloon has an inflated diameter substantially the same as the diameter of the sheath. In another embodiment, the balloon has an inflated diameter smaller than the diameter of the sheath.

The use of a balloon for the dilator tip provides a structure which can readily be inflated and deflated, via appropriate fluid supply through a lumen in the assembly.

Advantageously, said element forms a distal most part of the introducer assembly. Although embodiments are envisaged in which there may be a feature beyond the compressible element (such as a narrow point of the dilator tip), it is preferred that the compressible element is the endmost component of the assembly. The dilator tip is provided with a carrier, such as a cannula. The carrier may be an actuator for moving the dilator tip relative to the lumen of the implantable medical device. The carrier, in use, may extend from the dilator tip to outside of the patient. The dilator top may be withdrawn into the lumen by pulling on the carrier. The carrier may be pulled so that the entirety of the dilator tip is withdrawn into or through the lumen.

In an embodiment, the dilator tip is provided with at least one radial constriction on an outer surface thereof operable to increase bending flexibility of the dilator tip. Advantageously, the or each said radial constriction is formed as a portion of balloon having a smaller expanded diameter. These features give the dilator tip increased bending flexibility, useful in guiding the dilator tip smoothly through tortuous vasculature and other vessels.

The dilator tip may be provided with at least one radially arranged weakening feature which increases bending flexibility of the dilator tip.

Preferably, the element is expandable and/or contractible in stages in a longitudinal direction of the dilator tip. In an embodiment, the element is a balloon provided with selective inflation characteristics. The balloon may be provided with a plurality of inflatable compartments. The compartments may be separately inflatable.

This feature has the advantage of being able to constrain radially the dilator tip in stages as the introducer assembly reaches the treatment site, in the preferred embodiment to withdraw the dilator tip gradually. Thus, the assembly can still benefit from the provision of a dilator tip even as it gets very close to the treatment position.

In an embodiment the introducer assembly includes a dilator tip catheter having an internal diameter; the implantable medical device being located concentrically to and radially outside of said dilator tip catheter; the dilator tip catheter and the implantable medical device being located within said sheath; wherein the radially expandable and contractible element of the dilator tip has an expanded diameter substantially equal to the diameter of the sheath and a contracted diameter smaller than the internal diameter of said dilator tip catheter, said dilator tip being retractable when in the contracted condition into said dilator tip catheter.

The dilator tip is provided with a carrier, such as a cannula. The carrier may also extend through the dilator tip catheter to the outside of the patient. The dilator tip may be withdrawn into the dilator tip catheter by pulling back on the carrier. The carrier may be pulled back so that the entirety of the dilator tip is within the dilator tip catheter. Not forming part of the invention, there is also disclosed herein a method of deploying an implantable medical device by means of an introducer assembly including: a dilator tip; a sheath having a diameter; an implantable medical device located within said sheath and providing a lumen therewithin when located in said sheath; wherein the dilator tip is formed with a radially expandable and contractible element, wherein said element has an expanded diameter substantially equal to the diameter of the sheath and a contracted diameter smaller than the diameter of the lumen of said implantable medical device, said dilator tip being retractable when in the contracted condition into said lumen; the method including the steps of: providing the dilator tip in an expanded condition; locating endoluminally the distal end of the introducer assembly in a patient at or proximate a treatment site; contracting the dilator tip; withdrawing the dilator tip into the sheath; moving the sheath in a distal direction after withdrawal of the dilator tip; and deploying the implantable medical device from the introducer.

Preferably, the element of the dilator tip is a balloon, the method including the step of inflating said balloon to one of: a diameter substantially no greater than the diameter of the sheath; a diameter substantially the same as the diameter of the sheath; and a diameter less than the diameter of the sheath.

Advantageously, the method is performed during the implantation of a medical device at or proximate the heart of a patient.

### Brief Description of the Drawings

Embodiments of the present invention are described below, by way of example only, with reference to the accompanying drawings, in which:
Figure 1 is a schematic diagram in cross-section of an adult aorta showing a prior art deployment assembly fed intraluminally up to the heart;
Figure 2 is a schematic diagram in perspective of an embodiment of introducer assembly;
Figure 3 is an enlarged view of the distal end of the assembly of Figure 2;
Figure 4 is a view of the distal end of the assembly of Figure 3 with the dilator tip in a contracted configuration;
Figure 5 is a view of the distal end of the assembly of Figure 3 showing the dilator tip withdrawn into a sheath of the assembly;
Figure 6 is an enlarged cross-sectional view of the embodiment of introducer assembly of Figures 2 to 5;
Figures 7 to 9 are schematic diagrams of other embodiments of dilator tip; and
Figure 10 is a schematic diagram in cross-section of an adult aorta showing the embodiment of deployment assembly of Figures 2 to 6 fed intraluminally up to the heart.

### Description of the Preferred Embodiments

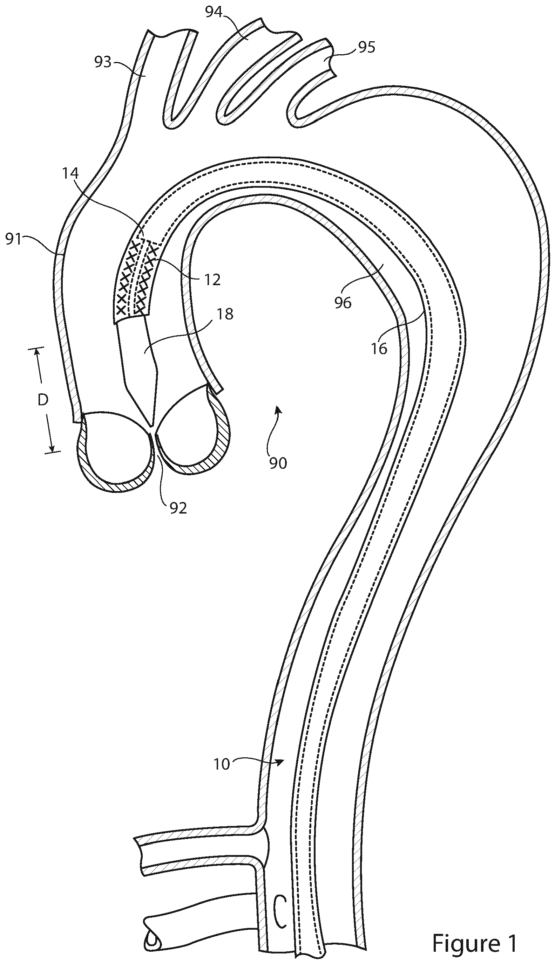

Referring to Figure 1, there is shown in cross-section an example of a thoracic aorta. It will be seen that the thoracic aorta 90 comprises an ascending aorta 91 which receives blood from the heart through an aortic valve 92. At the upper end of the ascending aorta there are branches for the great vessels, the innominate artery 93, the left common carotid artery 94 and the left subclavian artery 95. The aorta after these great vessels is referred to as the descending aorta 96.

Located within the aorta there is shown a prior art introducer assembly 10, which typically passes into the patient's vasculature through the femoral artery (not shown) and in this example through the aortic arch to a location just proximate the aortic valve 92. The introducer assembly 10 carries an implantable medical device 12, for instance a stent graft, within a holding sheath 16. The assembly typically includes a pusher member 14 for pushing the device 12 to the deployment site or otherwise for holding the device 12 within the introducer assembly during the deployment operation. There is also provided at the distal end of the assembly a dilator tip 18, of conventional form, which has the function of assisting in curving and guiding the assembly 10 through tortuous vasculature.

It is common for such assemblies 10 to include a guide wire (not shown) which passes through a central lumen of the assembly 10, as well as in the dilator tip 18, for use in guiding the assembly through the vasculature during the deployment operation. The dilator tip 18 is typically of a flexible material. Where such a guide wire is provided, there is generally little risk of this damaging the patient's vasculature or organs in light of the very flexible nature of the distal end of the guide wire. It is thus not uncommon for such systems to provide for the guide wire to enter the cardiac chamber through the valve 92. The dilator tip 18, on the other hand, is preferably kept clear of the valve 92, in other words it is preferred in some instances at least not to pass the dilator tip 18 through the valve 92. The tip is too large and relatively hard for this to be desirable. As a result, in some clinical applications at least, the introducer assembly should not be made to breach the position of the valve 92.

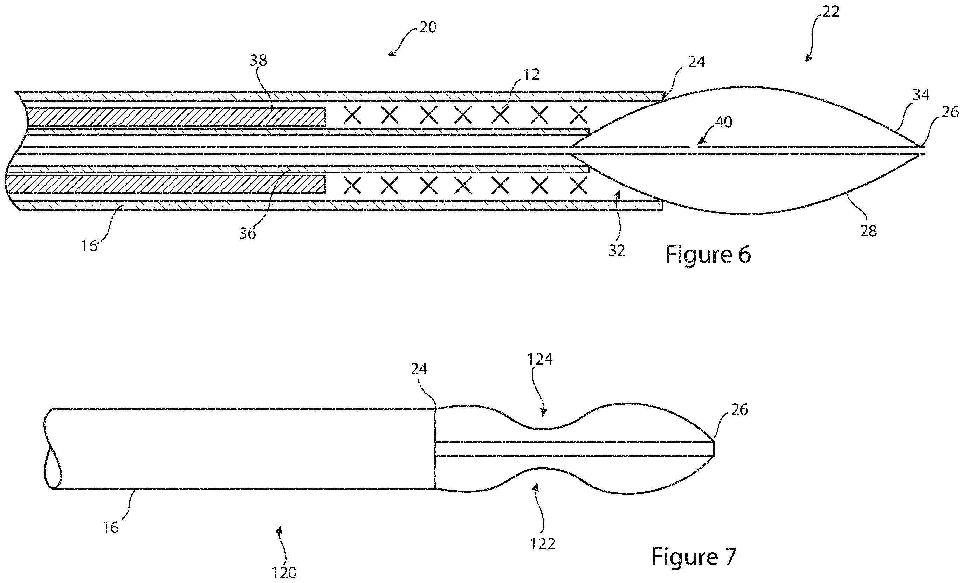

The dilator tip 18, which has a minimum effective length to give it the required flexibility, therefore limits the position at which the introducer can be moved towards the heart valve 92, typically to a gap or distance D as shown in Figure 1. Thus, the implantable medical device 12 will also only be able to be positioned in the vessel, prior to deployment, by no less than distance D from the aortic valve 92. In practice this distance may be significantly more than D if the dilator tip 18 must be extended out of the sheath 16 in order to clear space for release of the device 12. This gap D, therefore, means that the device 12 cannot be placed close to the heart. The invention is defined as in claim 1. 2 to 6 and 10 show an embodiment of assembly which can avoid this shortcoming of prior art devices. Figure 7 to 9 show other embodiments of inflatable dilator tip.

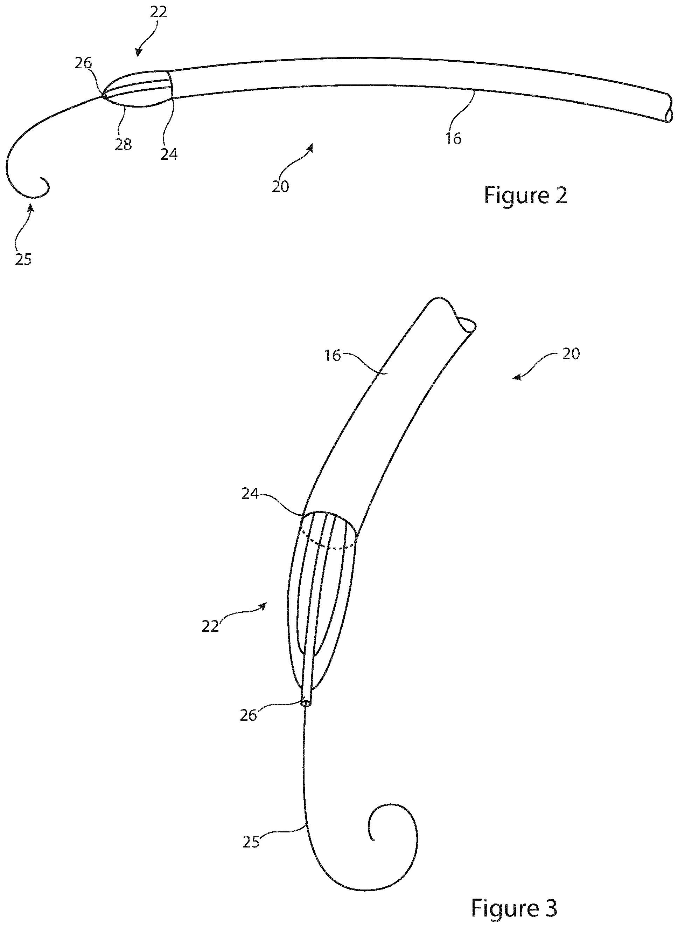

Referring first to Figures 2 and 3, these show in schematic form the distal end of a preferred embodiment of introducer assembly 20. The assembly includes a sheath 16 of conventional form and a dilator tip 22 which extends beyond the distal end 24 of the sheath 16. The dilator tip, formed with a radially expandable and contractible element, includes a flexible cannula 26 which extends along a longitudinal axis of the cannula and couples to a lumen in the introducer assembly 20, for the passage of a guide wire 25 through the assembly 20 and beyond the distal end of the dilator tip 22. The dilator tip also includes a balloon element 28, which is shown in inflated form in Figures 2 and 3.

As can be seen, shown also in Figure 6, the dilator tip 22 when inflated has an outer diameter at the junction with the distal end 24 of the sheath 16 which is similar to the outer diameter of the sheath 16. The diameter of the balloon 28 can be the same as, a little smaller or a little larger than the diameter of the sheath 16 at its distal end 24. In some embodiments, the balloon 28 of the dilator tip 22 may extend also into the lumen of the sheath 16 at its distal end 22, although in the preferred embodiment this is shaped in a manner similar to that of conventional dilator tips.

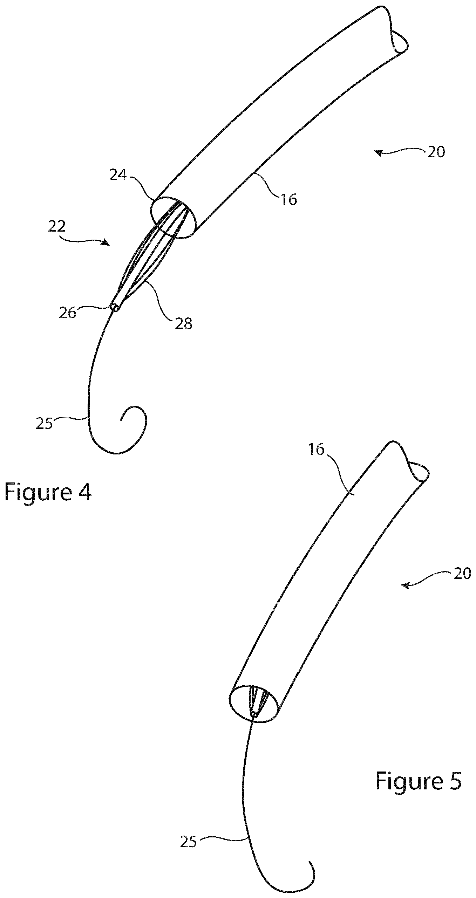

Referring now to Figure 4, the dilator tip 22 is shown in a deflated condition. In this condition the dilator tip has a diameter which is significantly smaller than the internal diameter of the sheath 16. As can be seen in Figure 5, in the deflated condition, the dilator tip 22 is able to be withdrawn into the sheath 16 and in practice through the lumen of the medical device carried in the sheath, as described in further detail below. Figures 4 and 5 also show guide wire 25 extending out of the cannula 26 of the dilator tip 22, as it typically would during the deployment procedure. What is relevant in these Figures, and as is explained in detail below, is that the introducer assembly 20 has a structure which enables the dilator tip to be withdrawn into the sheath 16 while the implantable medical device 12 is still located within the introducer and thus before deployment and release of the device.

Referring now to Figure 6, this is a cross-sectional view of a preferred embodiment of introducer assembly. The drawing is not to scale and shows the components of the assembly spaced from one another, not as they would be in practice but only for the sake of clarity.

Only the distal end of the assembly 20 is shown in Figure 6 as the components at its proximal end, that is at the external manipulation end of the assembly 20 and along its intermediate length, will be apparent to the skilled person having regard to the teachings herein.

The sheath 16 envelops the internal structure of the assembly 20 as well as the implantable medical device 12, and has the form of a conventional sheath. The dilator tip 22 has, in this embodiment, a shape when inflated which is analogous to that of a conventional dilator tip, with a proximal end 32 which extends a short distance into the sheath 16 and a distal end 34 which tapers.

Within the sheath 16 there is provided an implantable medical device 12. This may be any suitable implantable medical device, examples being stents, stent grafts and so on. There are typically provided within the introducer assembly elements for restraining the implantable medical device, such as restraining wires, holding sleeves and so on. The introducer assembly 20 can use any such device holding elements and therefore it is not necessary to describe any particular type of known restraining element herein.

The assembly also includes a pusher element 38, also of conventional form, which extends from a position at the end of the portion of the carrier catheter 36 which holds the implantable medical device 12 to a proximal end of the introducer assembly 20, typically at the external manipulation end. The pusher element 38 is also of tubular form having a central lumen therein typically for the passage therethrough of a guide wire and other elements of the introducer assembly. Typically, the pusher element 38 has an outer diameter substantially the same as or just smaller than the inner diameter of the sheath 16. The distal end of the pusher element 38, in this example, provides a shoulder for supporting the distal end of the implantable medical device 12 and may also be provided with at least one device restraining device or mechanism.

In the preferred embodiment, the medical device 12 lies within the sheath 16 in such a manner that is provides a lumen extending therewithin. The dilator tip 22 is able to pass within this lumen when in its deflated or contracted condition.

Figure 6 also shows an optional carrier cannula or catheter 36 which is able to accommodate the deflated balloon assembly 22. As can be seen, the optional carrier 26 sits within the bore of the pusher element 38 and can be useful in ensuring smooth retraction of the balloon dilator 22. It is envisaged that such a catheter 36 could also provide restraining mechanisms for restraining the medical device 12, if desired. A suitable restraining mechanism could include supports for holding one or more trigger wires for constraining the proximal and/or the distal end of the medical device 12.

The cannula 26 of the balloon assembly 22 extends within the lumen of the carrier catheter 36 and typically to the external manipulation end of the introducer assembly 20. The cannula 26 has a central lumen for receiving the guide wire 25, as will be apparent with reference to Figures 2 to 5 and the above description.

The cannula 26 also includes a lumen within its wall structure which ends at a port 40 within the volume of the balloon element 28, as will be apparent in Figure 6. This lumen and the port 40 are responsible for providing inflation fluid for inflating the balloon element 28 and also for deflating this when it is desired to withdraw the balloon element 28 into the catheter 36. The cannula may be pulled in a proximal direction to retract the dilation tip into the catheter.

The balloon element 28, in the example shown in Figure 6, includes a proximal portion 32 which extends partially into the sheath 16 and beyond the distal end 24, as well as a distal portion 34 which tapers towards the tip of the dilator element 22. It is preferred that the balloon element 28, when fully inflated, has a tapering shape towards its distal end which is similar to conventional dilator tips, although this is not essential.

When the balloon assembly 22 is in its inflated condition, the balloon 28 has an outer diameter which is about the same as, slightly smaller than or slightly larger than the outer diameter of the sheath 16, again in a manner which is not dissimilar from prior art dilator tips. The balloon element 28, however, can deflate to a size which enables it to be withdrawn into the cannula 36 during the deployment process. In use the cannula 26 is pulled back so as to withdraw the entirety of the dilator tip into the catheter 36. It will be appreciated that when the dilator tip 22 is deflated and withdrawn into the catheter 36, there is no element of the assembly 20 which extends beyond the distal tip of the distal end of the sheath 24 and, when the sheath 16 itself is withdrawn (that is, pulled back) the distal end of the carrier catheter 36 and the implantable medical device 12 form the distal most part of the assembly 20. This differs from prior art assemblies, in which the dilator tip remains the distal most part of the assembly during the deployment procedure and can only be withdrawn once the implantable medical device has been deployed, that is it has been expanded to its deployed state so as to provide a lumen or passage within the interior of the implantable medical device sufficiently large to be able to withdraw the dilator tip and any other components which are distal of the implantable medical device when this is constrained on the introducer assembly.

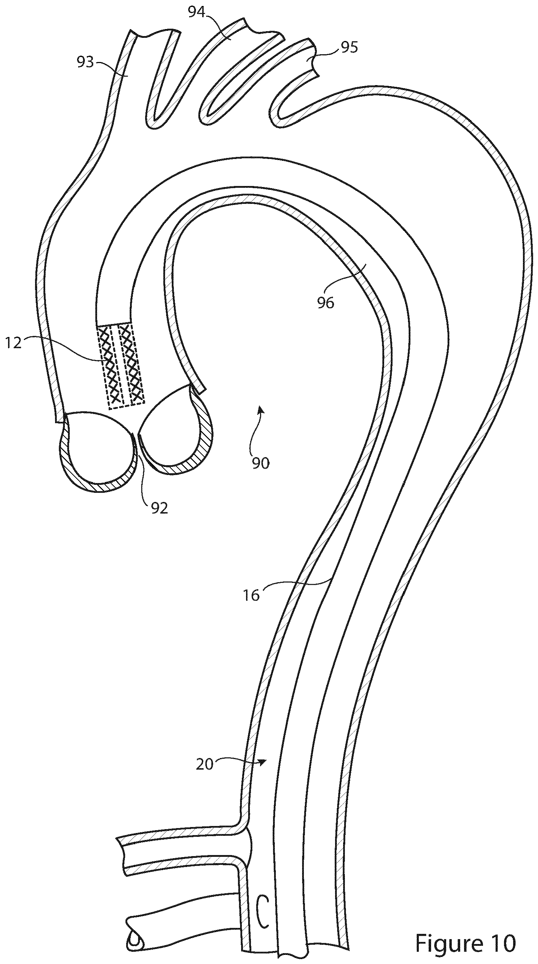

The structure of Figure 6 and of all of the embodiments envisaged in this patent application provides a dilator tip arrangement which can be constrained so as to be able to be withdrawn through the implantable medical device while this remains constrained on the introducer assembly and thus enables the implantable medical device to be located much closer to a desired deployment site, as will be described in further detail in connection with Figure 10.

Referring now to Figure 7, there is shown another embodiment of introducer assembly, the distal part only of this being shown in the Figure. The assembly is substantially the same as that shown in Figure 6 and of the other embodiments described in this application, the primary difference being the provision of a dilator tip, still of balloon form, in which the balloon element has at least one radial constriction on an outer surface thereof, in the preferred embodiment by virtue of having a waisted shaped when inflated. The narrow waist portion 124 of the balloon element 122 increases the radial flexibility (it is the flexibility in bending) of the balloon assembly 122 when this is inflated and therefore improves the ability of the balloon assembly 122 to curve so as to follow complex lumen anatomies.

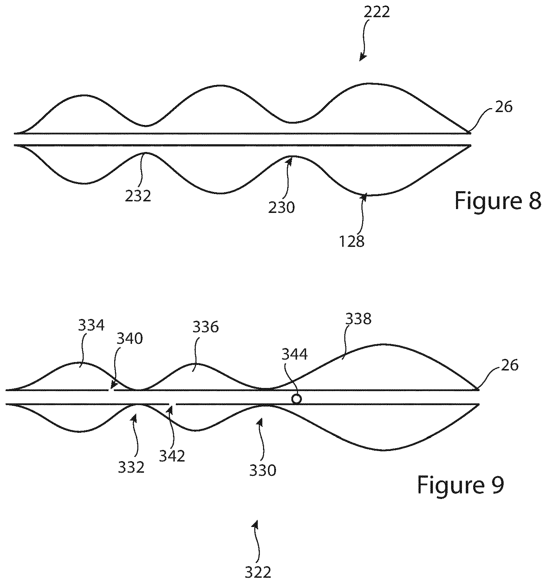

Figure 8 shows another embodiment of dilator tip 222, in which an inflatable balloon element 128 is provided with two waists 230, 232 in order to give the balloon 222 even greater curving flexibility.

Figure 9 shows an embodiment of dilator tip balloon assembly 322 which is equally provided with two waists of radial constrictions 330, 332 but in this case the waists 330, 332 are bonded or otherwise attached to the cannula 26 so as to create three separate chambers 334, 336, 338 to the balloon assembly 322. The cannula 26 is provided with three inflation/deflation lumens, each of which opens to a respective port 340, 342 and 344 located in respective balloon segments or chambers 334-338. The separate lumens and chambers enable the balloon assembly 322 to be deflated in stages. This would enable, for instance, the retention of a dilator tip portion during movement of the introducer assembly to the deployment site, for example very close to the aortic valve, while progressively deflating the most distal balloon segment so as to withdraw this into the catheter 36.

Referring now to Figure 10, there is shown the embodiment of introducer assembly shown in Figures 2 to 6 (although equally applicable to the other embodiments disclosed herein) located in the thoracic aorta of a patient and in a configuration in which the dilator tip balloon has already been withdrawn into the catheter element 36 and therefore is within the sheath 16. Figure 10 also shows the sheath 16 in two positions: (a) in dotted outline, in which the sheath is still overlying the implantable medical device 12, and (b) in solid lines, in which the sheath 16 has been withdrawn so as to expose the implantable medical device. It can be seen from Figure 10 that since the dilator tip 22 has been withdrawn into the assembly 20, the implantable medical device 12 can be located very close to the aortic valve 92, in this example, and thus much closer than is possible with prior art devices, a comparison of Figures 1 and 10 showing this quite clearly.

Although the preferred embodiments make use of an inflatable dilator tip, other embodiments are envisaged which use dilator tip of a different structure. The concept can be extended to any structure which is able to be radially compressed so as to be able to be withdrawn into the introducer assembly during the deployment operation, in the manner taught herein. Thus, the dilator tip could be of a compressible material which causes this to compress radially as it is being pulled into the inner catheter. In another embodiment, there could be provided another compressing mechanism, such as restraining wires arranged to close radially in on the tip so as to compress this. Other embodiments are also envisaged.

It will also be appreciated that although the preferred embodiments focus on an over the wire introducer system, the principles taught herein are equally applicable to introducer systems which do not use a guide wire. In such an event, it is not necessary to provide a cannula 26 within the dilator tip 22. The dilator tip could be provided with a flexible rod or wire or with no such central component, being made entirely of a balloon element at its distal end.

In place of a radial constriction such as the waist or waists shown in connection with the above described embodiments, the balloon could be provided with at least one radially arranged weakening feature which increases the flexibility of the dilator tip.

## Claims

1. An introducer assembly (20, 120) including:
a dilator tip (22, 222, 322) including a cannula having a central lumen for receiving a guidewire;
a sheath (16) having a diameter;
an implantable medical device (12) located within said sheath and providing a lumen therewithin when located in said sheath;
wherein the dilator tip is formed with a radially expandable and contractible element, **characterised in that** said element has an expanded diameter substantially equal to the diameter of the sheath and a contracted diameter smaller than the diameter of the lumen of said implantable medical device, said dilator tip being retractable when in the contracted condition into said lumen.

2. An introducer assembly according to claim 1, wherein the element is a balloon (28, 122, 128), said balloon having an inflated diameter no greater than the diameter of the sheath.

3. An introducer assembly according to claim 2, wherein the balloon (28, 122, 128) has an inflated diameter the same as the diameter of the sheath.

4. An introducer according to any preceding claim, wherein said element forms a distal most part of the introducer assembly.

5. An introducer assembly according to any preceding claim, wherein outer surfaces of the dilator tip are formed substantially entirely of said element.

6. An introducer assembly according to any preceding claim, wherein the dilator tip is provided with at least one radial constriction (124, 230, 232, 330, 332) on an outer surface thereof operable to increase bending flexibility of the dilator tip.

7. An introducer assembly according to claim 6, wherein the or each said radial constriction is formed as a portion of balloon having a smaller expanded diameter.

8. An introducer assembly according to any of claims 1 to 5, wherein the dilator tip is provided with at least one radially arranged weakening feature which increases bending flexibility of the dilator tip.

9. An introducer assembly according to any preceding claim, wherein the element is expandable and/or contractible in stages in a longitudinal direction of the dilator tip.

10. An introducer assembly according to any preceding claim, wherein the element is a balloon provided with selective inflation characteristics.

11. An introducer assembly according to claim 10, wherein said balloon is provided with a plurality of inflatable compartments (334, 336, 338).

12. An introducer assembly according to claim 12, wherein said compartments are separately inflatable.

13. An introducer assembly according to any preceding claim, including a dilator tip catheter (36) having an internal diameter; the implantable medical device (12) being located concentrically to and radially outside of said dilator tip catheter; the dilator tip catheter and the implantable medical device being located within said sheath (16); wherein the radially expandable and contractible element of the dilator tip has an expanded diameter substantially equal to the diameter of the sheath and a contracted diameter smaller than the internal diameter of said dilator tip catheter, said dilator tip being retractable when in the contracted condition into said dilator tip catheter (36).

14. An introducer assembly according to claim 13, wherein the dilator tip catheter includes at least one restraining mechanism for restraining a proximal and/or a distal end of the medical device.

## Patentansprüche

1. Einführeranordnung (20, 120), die Folgendes aufweist:
eine Dilatatorspitze (22, 222, 322), die eine Kanüle mit einem mittleren Lumen zur Aufnahme eines Führungsdrahts aufweist,
eine Hülse (16) mit einem Durchmesser, eine implantierbare medizinische Vorrichtung (12), die in der Hülse angeordnet ist und ein Lumen darin bereitstellt, wenn sie in der Hülse angeordnet ist,
wobei die Dilatatorspitze mit einem Element gebildet ist, das radial expandierbar und zusammenziehbar ist, **dadurch gekennzeichnet, dass** das Element einen expandierten Durchmesser hat, der im Wesentlichen gleich dem Durchmesser der Hülse ist, und
einen zusammengezogenen Durchmesser, der kleiner als der Durchmesser des Lumens der implantierbaren medizinischen Vorrichtung ist, wobei die Dilatatorspitze in das Lumen zurückziehbar ist, wenn sie im zusammengezogenen Zustand ist.

2. Einführeranordnung nach Anspruch 1, wobei das Element ein Ballon (28, 122, 128) ist, wobei der Ballon einen inflatierten Durchmesser hat, der nicht größer als der Durchmesser der Hülse ist.

3. Einführeranordnung nach Anspruch 2, wobei der Ballon (28, 122, 128) einen inflatierten Durchmesser hat, der derselbe wie der Durchmesser der Hülse ist.

4. Einführeranordnung nach einem der vorhergehenden Ansprüche, wobei das Element einen distalsten Teil der Einführeranordnung bildet.

5. Einführeranordnung nach einem der vorhergehenden Ansprüche, wobei Außenflächen der Dilatatorspitze im Wesentlichen vollständig aus dem Element gebildet sind.

6. Einführeranordnung nach einem der vorhergehenden Ansprüche, wobei die Dilatatorspitze an einer Außenfläche davon mit mindestens einer radialen Verengung (124, 230, 232, 330, 332) versehen ist, die dahingehend betätigbar ist, die Biegeflexibilität der Dilatatorspitze zu erhöhen.

7. Einführeranordnung nach Anspruch 6, wobei die oder jede radiale Verengung als ein Ballonabschnitt mit einem kleineren expandierten Durchmesser ausgebildet ist.

8. Einführeranordnung nach einem der Ansprüche 1 bis 5, wobei die Dilatatorspitze mit mindestens einem radial angeordneten Schwächungsmerkmal versehen ist, das die Biegeflexibilität der Dilatatorspitze erhöht.

9. Einführeranordnung nach einem der vorhergehenden Ansprüche, wobei das Element stufenweise in einer Längsrichtung der Dilatatorspitze expandierbar und/oder zusammenziehbar ist.

10. Einführeranordnung nach einem der vorhergehenden Ansprüche, wobei das Element ein Ballon ist, der mit gezielten Inflatierungseigenschaften versehen ist.

11. Einführeranordnung nach Anspruch 10, wobei der Ballon mit einer Vielzahl von inflatierbaren Fächern (334, 336, 338) versehen ist.

12. Einführeranordnung nach Anspruch 12, wobei die Fächer separat inflatierbar sind.

13. Einführeranordnung nach einem der vorhergehenden Ansprüche, die einen Dilatatorspitzenkatheter (36) mit einem inneren Durchmesser aufweist, wobei die implantierbare medizinische Vorrichtung (12) konzentrisch zu dem Dilatatorspitzenkatheter und radial außerhalb davon angeordnet ist, wobei der Dilatatorspitzenkatheter und die implantierbare medizinische Vorrichtung in der Hülse (16) angeordnet sind, wobei das radial expandierbare und zusammenziehbare Element der Dilatatorspitze einen expandierten Durchmesser hat, der im Wesentlichen gleich dem Durchmesser der Hülse ist, und einen zusammengezogenen Durchmesser, der kleiner als der innere Durchmesser des Dilatatorspitzenkatheters ist, wobei die Dilatatorspitze in den Dilatatorspitzenkatheter (36) zurückziehbar ist, wenn sie im zusammengezogenen Zustand ist.

14. Einführeranordnung nach Anspruch 13, wobei der Dilatatorspitzenkatheter mindestens einen Rückhaltemechanismus zum Rückhalten eines proximalen und/oder distalen Endes der medizinischen Vorrichtung aufweist.

## Revendications

1. Ensemble formant introducteur (20, 120) comprenant :
un embout de dilatation (22, 222, 322) comprenant une canule comportant une lumière centrale destinée à recevoir un fil-guide ;
une gaine (16) présentant un diamètre ;
un dispositif médical implantable (12) situé à l'intérieur de ladite gaine et formant une lumière à l'intérieur de celle-ci lorsqu'il est placé dans ladite gaine ;
l'embout de dilatation étant formé avec un élément radialement agrandissable et contractable, **caractérisé en ce que** ledit élément présente un diamètre agrandi essentiellement égal au diamètre de la gaine et un diamètre contracté inférieur au diamètre de la lumière dudit dispositif médical implantable, ledit embout de dilatation étant rétractable dans ladite lumière lorsqu'il se trouve dans l'état contracté.

2. Ensemble formant introducteur selon la revendication 1, dans lequel l'élément est un ballonnet (28, 122, 128), ledit ballonnet présentant un diamètre gonflé qui n'est pas supérieur au diamètre de la gaine.

3. Ensemble formant introducteur selon la revendication 2, dans lequel le ballonnet (28, 122, 128) présente un diamètre gonflé identique au diamètre de la gaine.

4. Introducteur selon l'une quelconque des revendications précédentes, dans lequel ledit élément forme une partie distale extrême de l'ensemble formant introducteur.

5. Ensemble formant introducteur selon l'une quelconque des revendications précédentes, dans lequel les surfaces extérieures de l'embout de dilatation sont formées essentiellement entièrement par ledit élément.

6. Ensemble formant introducteur selon l'une quelconque des revendications précédentes, dans lequel l'embout de dilatation est pourvu d'au moins un resserrement radial (124, 230, 232, 330, 332) sur une surface extérieure de celui-ci, servant à augmenter la souplesse en flexion de l'embout de dilatation.

7. Ensemble formant introducteur selon la revendication 6, dans lequel ledit resserrement radial ou chacun desdits resserrements radiaux est réalisé sous la forme d'une partie de ballonnet présentant un diamètre agrandi inférieur.

8. Ensemble formant introducteur selon l'une quelconque des revendications 1 à 5, dans lequel l'embout de dilatation est pourvu d'au moins un attribut d'affaiblissement disposé radialement qui augmente la souplesse en flexion de l'embout de dilatation.

9. Ensemble formant introducteur selon l'une quelconque des revendications précédentes, dans lequel l'élément est agrandissable et/ou contractable par étapes dans une direction longitudinale de l'embout de dilatation.

10. Ensemble formant introducteur selon l'une quelconque des revendications précédentes, dans lequel l'élément est un ballonnet pourvu de caractéristiques de gonflage sélectif.

11. Ensemble formant introducteur selon la revendication 10, dans lequel ledit ballonnet est pourvu d'une pluralité de compartiments gonflables (334, 336, 338).

12. Ensemble formant introducteur selon la revendication 12, dans lequel lesdits compartiments peuvent être gonflés séparément.

13. Ensemble formant introducteur selon l'une quelconque des revendications précédentes, comprenant un cathéter (36) d'embout de dilatation présentant un diamètre intérieur ; le dispositif médical implantable (12) étant placé de manière concentrique par rapport audit cathéter d'embout de dilatation et radialement à l'extérieur de celui-ci ; le cathéter d'embout de dilatation et le dispositif médical implantable étant situés à l'intérieur de ladite gaine (16) ; l'élément radialement agrandissable et contractable de l'embout de dilatation présentant un diamètre agrandi essentiellement égal au diamètre de la gaine et un diamètre contracté inférieur au diamètre intérieur dudit cathéter d'embout de dilatation, ledit embout de dilatation étant rétractable dans ledit cathéter (36) d'embout de dilatation lorsqu'il se trouve dans l'état contracté.

14. Ensemble formant introducteur selon la revendication 13, dans lequel le cathéter d'embout de dilatation comprend au moins un mécanisme de retenue servant à retenir un extrémité proximale et/ou distale du dispositif médical.
